# EUROPEAN PATENT APPLICATION

(11) **EP 3 693 974 A1**
(43) Date of publication of application: **12.08.2020**
(21) Application number: 19155911.1
(22) Date of filing: 07.02.2019
(51) Int. Cl.: G16H 20/10, G16H 20/40, G16H 50/30, C12Q 1/6886

(54) **IDENTIFYING RESPONSIVENESS TO RADIO-IMMUNO COMBINATION THERAPY**

(71) Applicant: Koninklijke Philips N.V., 5656 AG Eindhoven (NL)
(72) Inventor: RODRIGUES, Pedro, 5656 AE Eindhoven (NL); VITTORINO DE ALMEIDA, Vanda Lucia de Carvalho, 5656 AE Eindhoven (NL); WIMBERGER-FRIEDL, Reinhold, 5656 AE Eindhoven (NL)
(74) Representative: de Haan, Poul Erik

(57) **Abstract**

Described is a computer-implemented method for identifying responsiveness to therapy for a subject suffering from cancer. The method involves determining values of biomarkers such as the levels of TREX1, PD-L1 and/or immune cell infiltration in a cancerous lesion. The biomarker values are processed to obtain a score indicative of responsiveness of the cancerous lesion to combined radio- and immunotherapy treatment. The method may obtain scores for multiple treatment modalities (such as different doses and/or fractionations of radiotherapy) and/or different cancer lesions in the subject. Also provided is a system and computer program product for performing the method.

## Description

### FIELD OF THE INVENTION

Embodiments described herein generally relate to methods, systems and computer program products for identifying responsiveness to cancer therapy. The methods are particularly applicable to determining responsiveness to radiotherapy and immunotherapy combination treatments, and can be used to select specific treatment modalities for use in treating a subject. For instance, the method may be applied to determine a preferred dose and/or fractionation of radiotherapy to be used in combination with immunotherapy, and/or to select a preferred cancer lesion to be treated in the subject in order to produce an abscopal response.

### BACKGROUND OF THE INVENTION

Diagnostic and therapy decisions in oncology are largely based on the underlying biology. Understanding the mechanisms of the disease plays a key role in cancer research but can also help clinicians in making decisions and tracking the progress of the disease.

Radiation therapy (RT) has been used for more than a century and remains an effective treatment for local tumour control in the management of solid malignancies, with up to 50-60% of all cancer patients receiving such treatment (Harrington et al. (2011) Br. J. Cancer 105:628-39). Emerging evidence suggests that RT, in addition to its direct tumour cytotoxic effects, also stimulates specific immune responses which may play an important role in the overall process of RT-induced antitumor effects (Weichselbaum et al (2017) Nat. Rev. Clin. Oncol. 14(6):365-79). Thus, the optimal dose and fractionation for radiation, together with the mechanism of synergy for radiotherapy with immunotherapy, for the treatment of cancers is an active field of research.

Induction of adaptive immunity by RT is managed by the production of type I interferon (IFN) within the tumour micro-environment (TME), both in cancer cells and in antigen presenting dendritic cells, which activates the adaptive immune system and facilitates effective antigen cross-presentation and priming of tumour-specific CD8+ T-cells. However, the lack of a durable immune response to RT in established tumours is thought to be a consequence of an immuno-suppressive TME, which may contribute to disease recurrence and progression.

In addition, tumours with a highly suppressive TME for immune response and anomalies in the expression of certain markers are more at risk of not responding to combined radio-immunotherapy treatment.

Kosinsky et al (Journal for ImmunoTherapy of Cancer (2018) 6:17) describe a mathematical model to explore different radiation therapy and anti PD-L1 combination treatments that maximise anti-tumour responses. However, there are no known tools to assist a clinician with the selection of a successful therapy that will cause a tumour to respond to radiation in a way that stimulates the adaptive immune response using radiation in combination with immunotherapy (e.g. immune checkpoint inhibition).

### SUMMARY OF THE INVENTION

There is therefore a need for methods and systems that assist a clinician in identifying responsiveness of cancer lesions to radio-immunotherapy, and in particular to identify which therapy option will be most likely to be effective in a specific subject.

The object of the present invention is solved by the subject matter of the independent claims where further embodiments are incorporated in the dependent claims. It should be noted that the following described aspects of the invention apply equally to the method for identifying responsiveness to therapy for a subject suffering from cancer, to the system and to the computer program product.

In this context and according to one aspect, there is provided a computer-implemented method for identifying responsiveness to therapy for a subject suffering from cancer, the method comprising receiving data associated with a cancerous lesion in the subject; processing the data to determine a value for one or more biomarkers in the cancerous lesion; wherein the biomarkers comprise (i) an expression level of three prime repair exonuclease 1 (TREX1) in the cancerous lesion; an expression level of programmed death-ligand 1 (PD-L1) on tumor cells in the cancerous lesion; and/or (iii) a level of infiltration of immune cells in the cancerous lesion; processing the values of the biomarkers to obtain a score indicative of responsiveness of the cancerous lesion to one or more treatment modalities; wherein the treatment modalities comprise radiotherapy and immunotherapy combination treatment.

In one embodiment, the method comprises receiving data associated with a plurality of different cancerous lesions in the subject; processing the data to determine values for the biomarkers in each cancerous lesion; and processing the values of the biomarkers to obtain a score indicative of responsiveness of each cancerous lesion in the subject to the treatment modalities.

In another embodiment, the method comprises ranking each lesion based on the obtained scores, thereby indicating a lesion (or set of sub-lesions) in the subject to be selected for radiotherapy and immunotherapy combination treatment.

In another embodiment, the values of the biomarkers are processed to obtain a score indicative of responsiveness of the cancerous lesion to each of a plurality of treatment modalities; each treatment modality comprising a defined radiation dose and/or fractionation in combination with immunotherapy.

In another embodiment, the method comprises ranking each treatment modality based on the obtained scores, thereby indicating a radiation dose and fractionation to be selected for treatment of the subject in combination with immunotherapy.

In another embodiment, an increased value of the expression level of TREX1 in the cancerous lesion compared to a control value is processed to obtain a score indicative of reduced responsiveness of the lesion to radiotherapy and immunotherapy combination treatment, compared to immunotherapy alone.

In another embodiment, a reduced or unchanged value of the expression level of TREX1 in the cancerous lesion compared to the control value is processed to obtain a score indicative of increased responsiveness of the lesion to hypofractionated radiotherapy and immunotherapy combination treatment, compared to standard or single dose radiotherapy and immunotherapy combination treatment.

In another embodiment, an increased value of the expression level of PD-L1 on tumor cells and/or a reduced level of infiltration of immune cells in the tumor microenviroment compared to a control value is processed to obtain a score indicative of reduced responsiveness of the lesion to radiotherapy and immunotherapy combination treatment, compared to a lesion showing a lower value of the expression level of PD-L1 on tumor cells and/or an increased level of infiltration of immune cells.

In another embodiment, the method further comprises calculating an expected volume change of the cancerous lesion in response to the one or more treatment modalities.

In another embodiment, the received data is derived from RNA sequencing, polymerase chain reaction (PCR), microarray analysis or immunohistochemistry of a biopsy sample obtained from the subject.

In another embodiment, the immune cells comprise tumour infiltrating lymphocytes. Preferably the tumour infiltrating lymphocytes are CD8+, CD4+, FOXP3+ and/or CD3+ T cells.

In another embodiment, the immunotherapy comprises treatment with one or more immune checkpoint inhibitors, e.g. an anti-CTLA-4 (cytotoxic T lymphocyte associated protein 4) antibody such as ipilimumab, an anti-PD1 (programmed death 1) antibody such as nivolumab or pembrolizumab, or an anti-PD-L1 (programmed death ligand 1) antibody such as atezolizumab, durvalumab or avelumab.

In another embodiment, the received data is derived from positron emission tomography (PET) imaging of one or more of the biomarkers in the subject by means of a suitable radiotracer such as for example an antibody PD-L1 PET tracer (e.g. 89Zr-atezolizumab or 18F-BMS-986192 or an antibody against CD8+ lymphocytes (e.g. 89Zr-Df-IAB22M2C);

In another embodiment, the treatment modalities are stored in a treatment plan database; one or more treatment modalities are extracted from the database for analysis; each extracted treatment modality is analysed to determine a score indicative of responsiveness of the cancerous lesion to the treatment modality; and a treatment modality having a score indicative highest responsiveness is indicated to be selected for treatment of the subject.

In another aspect, there is provided a system for identifying responsiveness to therapy for a subject suffering from cancer, the system comprising an interface for receiving data associated with a cancerous lesion in the subject; a memory; and a processor configured to execute instructions stored on the memory to (a) process the data to determine a value for one or more biomarkers in the sample; wherein the biomarkers comprise i) an expression level of three prime repair exonuclease 1 (TREX1) in the cancerous lesion; (ii) an expression level of programmed death-ligand 1 (PD-L1) on tumor cells in the cancerous lesion; and/or (iii) a level of infiltration of immune cells in the cancerous lesion; and (b) process the values of the biomarkers to obtain a score indicative of responsiveness of the cancerous lesion to one or more treatment modalities; wherein the treatment modalities comprise radiotherapy and immunotherapy combination treatment.

In another aspect, there is provided a computer program product comprising a non-transitory computer readable medium, the computer readable medium having computer readable code embodied therein, the computer readable code being configured such that, on execution by a suitable computer or processor, the computer or processor is caused to perform the method as described herein.

### BRIEF DESCRIPTION OF THE DRAWINGS

Exemplary embodiments of the invention will now be described with reference to the following drawings wherein:
Figure 1 shows a systematic block diagram of a computerized system for identifying responsiveness to therapy of a subject suffering from cancer; and
Figure 2 shows a flow chart of a method for identifying responsiveness to therapy of a subject suffering from cancer.

### DETAILED DESCRIPTION OF EMBODIMENTS

The combination of radiotherapy with immunotherapy holds the promise of combatting cancer in the advanced setting for a larger group of patients. Described herein is an automated method that enables customisation of tumour treatment by determining responsiveness based on biomarker expression in the tumour, as well as the identification of e.g. metastatic tumours which should be selected for combined radiotherapy-immunotherapy. The analysis of responsiveness to possible radiotherapy treatment modalities available (for example standard, accelerated, hypo-fractionation, ablative high-dose) is also described.

Recent studies have demonstrated that hypofractionated radiotherapy can stimulate abscopal responses (the priming of immune cells against tumour antigens) more effectively than high-dose single fraction radiotherapy when combined with cytotoxic T lymphocyte antigen-4 (CTLA-4) immunotherapy. A possible explanation for such findings is the discovery that Type-I interferon (IFN-β) signalling originating from irradiated tumour cells is critical for recruitment and activation of BATF3-dependent dendritic cells to the tumour and subsequent radiotherapy-driven anti-tumour immunity (see for example Vanpouille-Box et al, "DNA exonuclease Trex1 regulates radiotherapy-induced tumour immunogenicity", Nature Communications, 8:15618 (2017); Yamazaki et al, "TREX1 Cuts Down on Cancer Immunogenicity", Trends in Cell Biology, August 2017, Vol. 27, No. 8:543-545; Diamond et al, "Exosomes shuttle TREX1-sensitive IFN-stimulatory dsDNA from irradiated cancer cells to DCs", Cancer Immunol Res. 2018 Aug;6(8):910-920). Induction of IFN-β by radiotherapy is dependent upon the sensing of cytosolic DNA in the tumour and dendritic cells via a signalling cascade involving cyclic GMP-AMP synthase (cGAS) and STimulator of INterferon Genes (STING). In tumour cells, this pathway has recently been shown to be attenuated by the DNA exonuclease three prime repair exonuclease 1 (TREX1) in a radiation dose-dependent manner. While single doses of radiation above 12-18 Gy induce TREX1, fractionated radiotherapy doses below a threshold to induce TREX1 amplify the production of IFN-β, leading to optimal activation of Batf3 transcription factor positive dendritic cells and priming of tumour-specific immunity (Vanpouille-Box et al, "Toward Precision Radiotherapy for Use with Immune Checkpoint Blockers", Clin Cancer Res. 2018 Jan 15;24(2):259-265; Deng et al, "STING-Dependent Cytosolic DNA Sensing Promotes Radiation-Induced Type I Interferon-Dependent Antitumor Immunity in Immunogenic Tumors", Immunity 2014, 41(5):843-852).

In particular, treatment with a radiation regimen that causes the accumulation of double strand break DNA (dsDNA) in the cytosol of cancer cells without inducing TREX1 activates the interferon type-I pathway via cGAS/STING. Downstream recruitment of BATF3-dependent dendritic cells and activation of CD8+ T cells is enabled, and tumour rejection occurs in synergy with anti-CTLA4 or anti-PD-L1 antibodies. In contrast, in a tumour treated with a dose of radiation above the threshold for TREX1 induction, dsDNA is cleared from the cytosol precluding IFN-β release by the cancer cells. This leads to insufficient dendritic cell recruitment and activation and lack of CD8+ T cell activation, resulting in the absence of local and abscopal tumour regression in combination with immune checkpoint inhibitors.

However, over-expression of TREX1 in tumour cells has been reported (Tomicic, M.T., Aasland, D., Nikolova, T., Kaina, B. and Christmann, M. (2013) Human three prime exonuclease TREX1 is induced by genotoxic stress and involved in protection of glioma and melanoma cells to anticancer drugs. Biochim. Biophys. Acta, Mol. Cell Res. 1833(8): 1832-1843; Christmann et al, "Three prime exonuclease I (TREX1) is Fos/AP-1 regulated by genotoxic stress and protects against ultraviolet light and benzo(a)pyrene-induced DNA damage" Nucleic Acids Res. 2010 Oct;38(19):6418-32), providing certain metastatic lesions with an adaptation mechanism to bypass the effects of DNA-damage caused by chemotherapy drugs. Therefore, lesions that exhibit such up-regulation may show a very effective degradation of cytosol dsDNA produced by radiotherapy, avoiding the cGAS/STING activation.

In itself, loss of STING and/or cGAS expression has also been reported in over a third of colorectal cancers (Xia et al, "Deregulation of STING Signaling in Colorectal Carcinoma Constrains DNA Damage Responses and Correlates With Tumorigenesis" Cell Rep. 2016 Jan 12;14(2):282-97). STING has also been shown to be frequently inactivated in HPV+ cancers, around 20% in ovarian cancer (Queiroz et al, "Defective STING signaling in ovarian cancer cells favor oncolytic virus action" J Immunol May 1, 2017, 198 (1 Supplement) 130.28), and in a significant portion of primary and metastatic melanoma and non-small cell lung cancer (NSCLC) (Caldwell et al, "Differential Expression of IFN-Stimulating DNA Sensors STING and cGAS in Lung Cancer Subtypes", MA 05.14, Journal of Thoracic Oncology Vol. 12 No. 11S2, Pages S1819-S1820 2017) . In general STING expression is decreased in tumour tissues and can be lost as the tumour progresses (Konno et al, "Suppression of STING signaling through epigenetic silencing and missense mutation impedes DNA damage mediated cytokine production", Oncogene. 2018 Apr;37(15):2037-2051, Song et al "Decreased expression of STING predicts poor prognosis in patients with gastric cancer", Sci Rep. 2017; 7: 39858). For example, in NSCLC stage IV, 60% of the malignant lesions showed no STING expression, correlating with an observed decreased survival for patients lacking STING expression.

Patients with gain-in-function TREX1 (over-expression), low cGAS/STING expression and low tumour lymphocytes (CD8+) infiltration at baseline are therefore less suitable for combined radiation and immunotherapy (e.g. using immune checkpoint inhibitors) and benefit more from other therapies. Alternatively, the addition of a drug that targets the source of the STING transcriptional inhibition or induces the down-regulation of TREX1 could be required.

Also in the context of radiotherapy, Programmed death-ligand 1 (PD-L1) up-regulation is a mechanism by which tumours may escape the adaptive immune response. Early identification of tumours that are more prone to exhibit such mechanism is important to enable selection of an appropriate anti-PD1/anti-PD-L1 therapy with the correct timing with respect to radiotherapy.

In parallel to the current functionality of TREX1 and cGAS/STING, the tumour microenvironment (TME) also plays a critical role in supporting the rationale for radio-immunotherapy combinations. TMEs that are characterised by existing CD8+ infiltration at baseline are less dependent on the correct TREX1/cGAS/STING functionality. Tumours that exhibit a supportive TME, as well as a correct function of TREX1 and cGAS/STING, will likely be the best responders to combined therapy. On the other hand, tumours that have a TME highly suppressive of the immune response, for example by checkpoint up-regulation (PD-L1), and large hypoxic regions are more dependent on the correct TREX1 and cGAS/STING functionality. Tumours with a highly suppressive TME for immune response and TREX1 and/or cGAS/STING expression anomalies are more at risk of not responding to combined radio-immunotherapy treatment.

In the setting of advanced (metastatic) disease, variability between tumours can lead to differences in the TREX1, Tumour Infiltrating Lymphocytes (TILs) and checkpoint expression (e.g. PD-L1). Therefore, assessment of relevant biological pathways derived from tissue of different tumours and/or *in vivo* imaging is helpful to select those tumours (as well as the ideal radiation dose and fractionation schemes) that could be irradiated in order to generate a robust response to the therapy.

By taking these biomarkers into account, tumour treatment may be adapted to the individual patient. For example, TREX1 over-expression provides extra protection against double strand DNA break therapeutics (e.g. chemotherapy and radiotherapy). Metastases that have over-expression of TREX1, low tumour infiltrating lymphocytes (TILs) and high PD-L1 expression are less likely to trigger expression of IFN-β and damp the overall immune response that follows radiotherapy and therefore are not the most suitable target for radiation therapy.

Various embodiments are described more fully below with reference to the accompanying drawings, which form a part hereof, and which show specific exemplary embodiments. However, the concepts of the present disclosure may be implemented in many different forms and should not be construed as limited to the embodiments set forth herein; rather, these embodiments are provided as part of a thorough and complete disclosure, to fully convey the scope of the concepts, techniques and implementations of the present disclosure to those skilled in the art. Embodiments may be practiced as methods, systems or devices. Accordingly, embodiments may take the form of a hardware implementation, an entirely software implementation or an implementation combining software and hardware aspects. The following detailed description is, therefore, not to be taken in a limiting sense.

Reference in the specification to "one embodiment" or to "an embodiment" means that a particular feature, structure, or characteristic described in connection with the embodiments is included in at least one example implementation or technique in accordance with the present disclosure. The appearances of the phrase "in one embodiment" in various places in the specification are not necessarily all referring to the same embodiment.

Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices. Portions of the present disclosure include processes and instructions that may be embodied in software, firmware or hardware, and when embodied in software, may be downloaded to reside on and be operated from different platforms used by a variety of operating systems. The invention may take form in various components and arrangements of components, and in various process operations and arrangements of process operations.

The present disclosure also relates to an apparatus for performing the operations herein. This apparatus may be specially constructed for the required purposes, or it may comprise a general-purpose computer selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each may be coupled to a computer system bus. Furthermore, the computers referred to in the specification may include a single processor or may be architectures employing multiple processor designs for increased computing capability.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any particular programming language that is sufficient for achieving the techniques and implementations of the present disclosure may be used. A variety of programming languages may be used to implement the present disclosure as discussed herein.

In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein. Numerous additional advantages and benefits will become apparent to those of ordinary skill in the art upon reading the following detailed description.

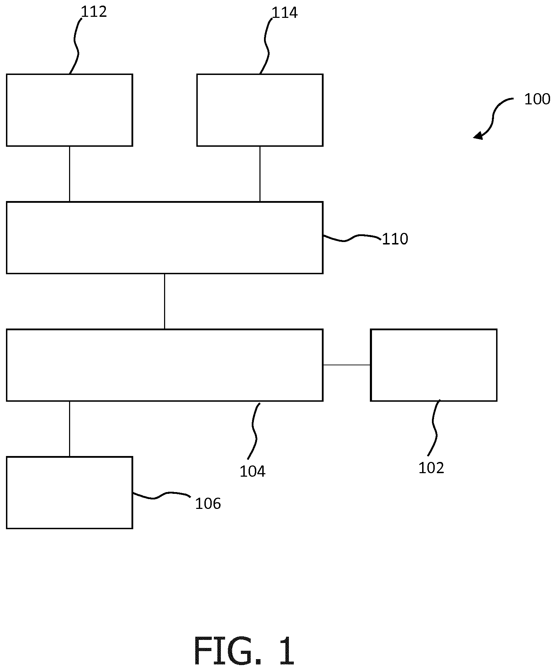

FIG. 1 illustrates a system 100 for identifying responsiveness to therapy a subject suffering from cancer in accordance with one embodiment.

The system 100 may include a user input/output (I/O) device 102 and a processor 104 executing instructions stored on memory 106. The processor 104 may be in communication with or otherwise include an interface 110 for receiving data from data sources 112 and 114. The data is associated with a cancerous lesion in the subject, e.g. the data may be derived by analyzing a biopsy sample from the subject or by *in vivo* functional imaging of the subject. Thus data sources 112 and 114 may comprise, for example, a microscopy imaging system, such as a digital microscope. Alternatively, data sources 112 and 114 may comprise a molecular analysis device such as automated nucleic acid sequencer or a microarray. In other embodiments, the data source may comprise a functional or radiological imaging system such a positron emission tomography (PET) scanner, a magnetic resonance imaging (MRI) scanner, computerized tomography (CT), and/or single-photon emission computed tomography (SPECT). The value determined from the PET image, specific to the used PET tracer, may reflect PD-L1 levels (e.g. 89Zr-atezolizumab, 18F-BMS-986192; estimate of the presence of CD8+, CD4+ tumor-cell infiltrating lymphocytes (e.g. 89Zr-Df-IAB22M2C).

The I/O device 102 may be any suitable device that can receive commands from an operator and output processed data graphically and/or in any other form. The I/O device 102 may be configured as, for example but without limitation, a personal computer, a tablet, laptop, mobile device, or the like.

The processor 104 may be any specifically configured processor or hardware device capable of executing instructions stored on memory 106 to process biological data in order to determine quantitative values therefrom. The processor 104 may include a microprocessor, a field programmable gate array (FPGA), application-specific integrated circuit (ASIC), or other similar device. In some embodiments, such as those relying on one or more ASICs, the functionality described as being provided in part via software may instead be hardwired into the operation of the ASICs, and as such, any associated software may be omitted.

The memory 106 may be L1, L2, L3 cache or RAM memory configurations. The memory 106 may include non-volatile memory such as flash memory, EPROM, EEPROM, ROM, and PROM, or volatile memory such as static or dynamic RAM, as discussed above. The exact configuration/type of memory 106 may of course vary as long as instructions for analyzing biological data can be executed by the processor 104.

The interface 110 may receive biological data from the data sources 112 and/or 114. The interface 110 may then communicate the received data to the processor 104 for analysis. The biological data may be, for example, in the form of digital microscopic images of a biopsy sample derived from a cancerous lesion in the subject. Alternatively, the biological data may be in the form of nucleic acid sequencing data, e.g. derived from RNA sequencing of the biopsy sample. In another embodiment, the biological data may be in the form of digital images obtained by radiological (e.g. PET) imaging of the subject.

The processor 104 is configured to calculate a value for one or more biomarkers in the sample, based on the received biological data. For example, the processor may calculate expression levels of TREX1 and/or PD-L1 in the sample, and/or a level of infiltration of immune cells in the cancerous lesion. The processor 104 then calculates a score indicative of responsiveness of the cancerous lesion to one or more treatment modalities. The treatment modalities typically comprise varying doses and fractionation of radiotherapy in combination with immunotherapy.

After analysis of the received data, the processor 104 may output the obtained scores indicative of responsiveness to the treatment modalities to the I/O device 102 or another display unit. In some embodiments, the processor may rank different lesions in the subject according to their suitability for combination radio-immunotherapy, and output an indication of a lesion or sub-set of lesions to be selected for treatment. In other embodiments, the processor may rank each treatment modality based on suitability to treat a specific cancer lesion, and output a particular treatment modality (e.g. radiation dose and fractionation) to be selected for treating the subject in combination with immunotherapy. The output may be in the form of a list of scores, a ranking table, a treatment recommendation, or e.g. a graphical representation of any of the above.

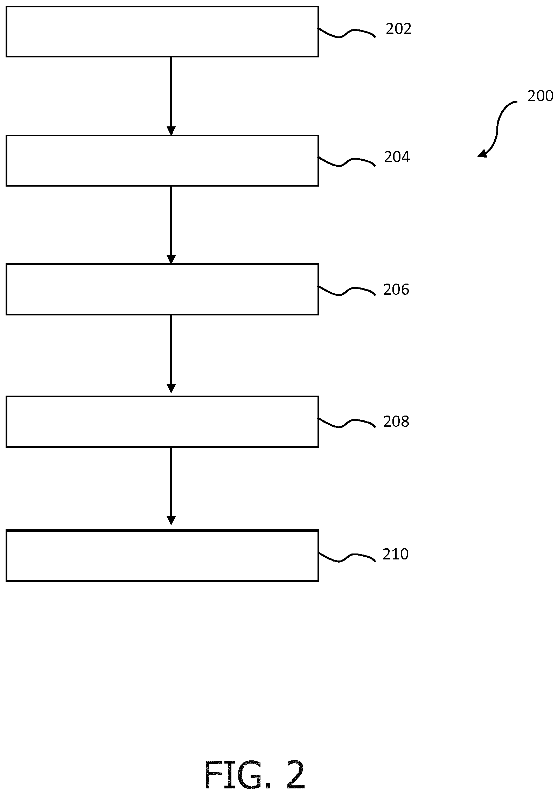

FIG. 2 depicts a flowchart of a method 200 for identifying responsiveness to radio-immunotherapy in a subject using the system of FIG. 1 in accordance with one embodiment. Step 202 involves receiving data associated with a biopsy sample derived from a cancerous lesion in the subject. The data (e.g. digital microscopic images) may be received by the interface 110 from the data sources 112 and/or 114. A processor such as the processor 104 of FIG. 1 may receive these images from the interface 110 of FIG. 1. In alternative embodiments, the biological data may be transmitted to the interface 110 and/or processor 104 by the I/O device 102, e.g. where the biological data is stored in another location after data acquisition by the data sources.

Step 204 involves processing the data to determine a value for one or more biomarkers in the sample. For instance, the processor 104 may use an algorithm suitable for analysis of digital microscopy images to identify stained objects within the tissue. In one embodiment, the biopsy sample may have been processed by immunohistochemistry using an antibody directed against one of the biomarkers, e.g. an anti-PD-L1 antibody. In such embodiments, the processor may detect staining in the image indicative of PD-L1 expression. Alternatively, the processor may analyse the image to identify a subset of cells such as cancer cells and/or immune cells (e.g. tumor infiltrating lymphocytes). Individual cell types may be identified based on expression of characteristics markers, e.g. protein expression or characteristic cell nuclei morphology. The processor quantifies the level of staining and/or the number of infiltrating immune cells in order to determine a value for the expression level.

Step 206 involves processing the values of the biomarkers obtained in step 204 to obtain a score indicative of responsiveness of the cancerous lesion to one or more treatment modalities. For instance, the processor may access a treatment plan database containing a plurality of treatment modalities, and extract potential treatment modalities for the subject therefrom. Each treatment modality may comprise a defined dose and/or fractionation of radiotherapy to be used in combination with immunotherapy for treating cancer. For each treatment modality, the processor calculates the responsiveness score based on the values of the biomarkers obtained in step 204.

In some embodiments, step 208 involves ranking the treatment modalities based on the responsiveness scores obtained in step 206. For instance, the processor 104 may analyse the obtained responsiveness scores in order to identify a treatment modality (e.g. dose and fractionation of radiotherapy for use in combination with immunotherapy) having a score indicative of highest responsiveness.

Step 210 involves providing an output via the I/O device 102 or a display to a user, e.g. a clinician. The output of the method may comprise, for example, a list of responsiveness scores for each treatment modality. The responsiveness scores may be ranked in order to indicate a treatment modality that is likely to be most effective in treating the cancerous lesion in the subject. Thus in some embodiments, the output may comprise a treatment recommendation to the clinician, e.g. a dose and fractionation of radiotherapy to be provided to the subject.

The steps of the method shown in Fig. 2 are now described in more detail below. The methods described herein involve (e.g. in step 202 shown in Fig. 2) a step of receiving data associated with a biopsy sample derived from a cancerous lesion in a subject. The received data may be, for example, a microscopy image such as a digital light microscopy image, e.g. obtained by a digital microscope. The image typically shows a magnified view of the tissue from the biopsy sample, that has been processed to stain for one or more of the biomarkers.

The tissue specimen (e.g. obtained by biopsy from a subject) can be prepared using known techniques for light microscopy analysis and imaging. For instance, haemotoxylin and eosin (H&E) staining of paraffin-embedded sections is the default technology to visualize tissue on a glass slide for pathology analysis. Immunohistochemistry (IHC) staining is a well-known approach to identify overexpression of proteins on cells in pathology tissue slides. The staining results in a typical brown appearance of tissue where the targeted protein is overexpressed as compared to normal. For example, by using an antibody against programmed death ligand 1 (PD-L1), its overexpression can be detected. The result is typically indicated as a so-called proportionality score, i.e. the percentage of tumor cells that are designated as positive (above threshold).

In other embodiments, the received data may comprise (e.g. as an alternative to, or in addition to data associated with a biopsy sample such as pathological and/or immunohistochemical results) data (such as one or more images) obtained by radiological methods and/or *in vivo* functional imaging of the subject. For instance, the data (e.g. image) may be obtained by positron emission tomography (PET), computerized tomography (CT) and/or single-photon emission computed tomography (SPECT). In one embodiment the data is a PET image. However in other embodiments, the image may be derived from alternative medical imaging methods such as magnetic resonance (MR) imaging.

For instance, the received data may be obtained by functional *in vivo* PET imaging, e.g. anti-CD8 PET imaging, anti-PD1/PDL-1 PET imaging or hypoxia PET imaging. Such methods may be used to score the degree by which the tumour micro-environment of a particular lesion will favour combined radio- and immunotherapy treatment. As an example, functional imaging of PD-L1 expression may be obtained by anti-PD1/PD-L1 PET imaging.

In step 204 of the method, biomarkers present in the cancerous lesion (as present e.g. in the biopsy sample or *in vivo* in the subject) are detected and quantified by analyzing the provided data. The detection of features in pathology slides may be performed using known computer algorithms which can analyse digital images of the slides. For instance, convolutional neural networks may be trained by providing annotated data sets of pathology images where the objects of interest have been annotated manually by a pathologist. Such objects can be for instance cell nuclei. Also the classification of cell nuclei into for instance tumor cells or immune cells can be trained successfully. Thus in particular embodiments, deep learning computer algorithms may be trained and/or used to detect cell nuclei on digital images of tissue, and even discriminate between tumor and non-tumor tissue.

Computer-based detection algorithms may also be used in combination with IHC to detect cells of interest (e.g. overexpressing a particular protein) automatically. For instance, the presence and abundance of cells that are classified as being positive for the overexpression of PD-L1 in IHC images can be determined. Using computer-based detection enables true quantification of the number and density of objects in a region of interest, e.g. the level of infiltration of immune cells into a tumor lesion.

Various biomarkers may be analysed and quantified in step 204, depending on how the biopsy sample has been processed and the data which has been received in step 202. In one embodiment the biomarkers may comprise an immune checkpoint molecule such as programmed death-ligand 1 (PD-L1), or a complementary set of biomarkers (such as cyclic GMP-AMP synthase (cGAS) and STimulator of INterferon Genes (STING)) or a gene signature panel. In one embodiment, statistically insignificant or no expression of one or more immune checkpoint molecules (e.g. PD-L1) may inform a positive treatment outcome or prediction for combined radiation and immunotherapy. For instance, a reduced value of the expression level of PD-L1 on tumor cells in the sample (e.g. compared to a control value indicative of a sample of known responsiveness) may be processed in step 206 to obtain a score indicative of increased responsiveness to combined radio- and immunotherapy.

In one embodiment, the biomarker is a level of infiltration of immune cells in the cancerous lesion. The immune cells may be, for example, tumour infiltrating lymphocytes (TILs), such as CD8+ T cells. Alternative or additional TILs include CD4+, FOXP3+ and/or CD3+ T cells. In one embodiment, a statistically significant level of TILs informs a positive treatment outcome or prediction. As an example, the degree of infiltration may be determined from data (e.g. a digital microscope image) derived from a Haemotoxylin and Eosin (H&E) stained biopsy sample from the cancerous lesion, or e.g. from molecular analysis (e.g. RNA sequencing).

In another embodiment, the biomarker is an expression level of three prime repair exonuclease 1 (TREX1) in the biopsy sample. The expression of TREX1 may be obtained from e.g. data derived from a molecular analysis of the sample, for instance using whole transcriptome shotgun sequencing (RNA sequencing or RNAseq), PCR, micro-array or nanoString-based methods. Typically statistically insignificant or no detection of constitutive up-regulation of TREX1 informs a positive treatment outcome or prediction. Thus in one embodiment, a reduced or unchanged value of the expression value of TREX1 in the sample compared to a control value (e.g. associated with a sample from a subject with known responsiveness) may be processed to obtain a score indicative of increased responsiveness of the lesion to combined radio- and immunotherapy, particularly hypofractionated radiotherapy and immunotherapy.

In another embodiment, the biomarker is a level of double strand-break DNA (dsDNA) in the cytosol of cells obtained from the lesion. In one embodiment, accumulation of cytosolic dsDNA informs a positive treatment outcome or prediction for combined radiation and immunotherapy. For instance, an increased value of dsDNA in the lesion may be translated to a score indicative of increased responsiveness to combined radio- and immunotherapy.

In step 206 of the method, the values of the biomarkers obtained in step 204 are processed to obtain a score indicative of responsiveness to one or more treatment modalities. The treatment modalities comprise radiotherapy and immunotherapy combination treatment. For instance, various doses and/or fractionations of radiotherapy to be used in combination with immunotherapy may be analysed to obtain a responsiveness score in the subject based on the levels of biomarkers.

Radiotherapy may be given as a single non-fractionated dose of radiation. Alternatively, the radiotherapy may be fractionated, hyperfractionated (or superfractionated) or hypofractionated. Hypofractionated radiotherapy is a treatment schedule in which the total dose of radiation is divided into large doses and treatments are given once a day or less often. In contrast, hyperfractionated radiotherapy is a treatment schedule in which the total dose of radiation is divided into small doses and treatments are given more than once a day. Thus "fractionation" of radiotherapy as used herein typically refers to a timing schedule of administration of the treatment.

Radiotherapy may be given in combination with immunotherapy. In one embodiment, a responsiveness score for each of a plurality of treatment modalities, such as radiotherapy type, radiotherapy fractionation, immunotherapy type and immunotherapy sequence may be determined. The immunotherapy may be, for example, treatment with one or more immune checkpoint inhibitors, e.g. an anti-CTLA-4 (cytotoxic T lymphocyte antigen 4) antibody, an anti-PD1 (programmed death 1) antibody or an anti-PD-L1 (programmed death ligand 1) antibody. Suitable checkpoint inhibitors include ipilimumab, nivolumab, pembrolizumab, durvalumab, atezolizumab and avelumab. In an example, available (relevant) treatment options may be extracted from a clinical information system, e.g. a system that includes a database comprising a plurality of treatment plans.

The responsiveness score may be calculated in various ways, depending on e.g. which biomarkers are quantified and which treatment modalities are selected. For instance, in one embodiment the responsiveness score may be inversely proportional to a level of double strand break DNA (dsDNA) when TREX1 is over-expressed. Alternatively, the responsiveness score may be inversely proportional to the dsDNA level in a case where TREX1 expression is activated by a dose of radiation over a threshold for stimulating TREX1 expression. Alternatively, the responsiveness score may be proportional to the dsDNA level when TREX1 expression is below a threshold stimulated by a dose of radiation. Alternatively, the responsiveness score may be proportional to the operational status of the cGAS/STING pathway.

In one embodiment, an algorithm may be used, which is based on the sum of the individual scores between 0 and 1 attributed to each specific biomarker level measured in the sample of the subject. A positive treatment outcome may then be predicted if the sum reaches a certain threshold. Optionally or in addition, the weight of each individual biomarker score may be adjusted in order to improve the performance of the algorithm. In one embodiment, the responsiveness score may be computed as an expected change in lesion volume as function of treatment.

The system and method described herein is based on a score that integrates biomarker data derived from biopsy and/or functional imaging. For each radiation dose regime/fractionation and tumour, a score is computed. For a successful therapy using radiation in combination with immunotherapy (e.g. using immune checkpoint inhibitors), it is helpful to select a target tumour which will respond to radiation in a way that stimulates the adaptive immune response and also triggers a response in the abscopal tumours.

Thus in one embodiment, the method is used to rank metastatic lesions in an individual subject for responsiveness to combined radio- and immunotherapy, e.g. in order to select a lesion most likely to produce an abscopal effect. In a first step tumour tissue is acquired from at least one cancerous lesion. For example, the subject visits a biopsy laboratory where a biopsy procedure is performed to extract a tissue sample. The biopsy procedure can be a surgical procedure (incisional biopsy) or can employ a biopsy needle or other interventional instrument.

In some embodiments employing an interventional instrument, the biopsy procedure uses a hollow needle that is pushed into the tumour to extract a biopsy "core" whose dimensions correspond to a hollow interior of the biopsy needle. In this approach the structural integrity of the sample is preserved. In other embodiments, the biopsy procedure is a needle aspiration biopsy procedure typically used to extract a fluid or gelatinous tissue sample having limited structural integrity. The resulting extracted tissue is processed to generate one or more biopsy samples for analysis. In the case of a biopsy core or incised tissue sample, this typically entails sectioning the extracted tissue so as to generate a number of biopsy samples for analysis. For example, a biopsy core can be sectioned at multiple placed along the core so as to generate a line of biopsy samples extending along the length of the biopsy core (i.e., along the length of the biopsy needle as placed during tissue extraction). By pressing the needle into the tumour, the resulting line of biopsy samples are acquired at a range of depths into the tumour, which can be useful for assessing any inhomogeneity of the tumour. Preferably a number of biopsy samples are obtained, each from a different tumour lesion in the subject.

The tumour tissue is then assessed for:
a) Expression of TREX1 (detection of constitutive up-regulation of TREX1);
b) PD-L1 expression on tumour cells; and
c) Degree of infiltration of immune cells in tumour (TIL status).

Information obtained from the assessment is loaded into a decision support system, optionally with possible treatment configurations including radiotherapy type and fractionation, immunotherapy type and sequence. The decision support system computes a responsiveness score based on the input which is used to rank the tumours in terms of likelihood of expected overall treatment response. A resulting ranked list of tumours is presented to the user for the treatment.

Thus, in one example, the method and system described herein uses biomarkers obtained from a biopsy as an input and provides an output based on three main elements:
1) systemic tumour antigen presentation in concert with dendritic cell maturation as function of TREX1 activity status, radiotherapy dose and fraction for each tumour. In one embodiment, the resulting score will be inversely proportional to dsDNA in tumour if TREX1 over-expression is present. In another embodiment, the resulting score will be inversely proportional to dsDNA in tumour if TREX1 is activated due to radiation dose above a certain threshold per fraction. In an alternative embodiment, the resulting score will be proportional to generated dsDNA if dose fractionation is below TREX1 threshold. Expressed in another way, the score will be proportional to cGAS/STING pathway operational status.
2) T cells infiltration of a tumour at baseline, i.e. TILs densities; and
3) immune checkpoint molecules at baseline, e.g. negative/positive PD-L1 expression (percentage of PD-L1 positive tumour cells).

An example of the basic set of rules based on these biomarkers is shown in Table 1 :

**Table 1**

| Preferred Treatment Type | TREX1 | TIL | PD-L1 |
|---|---|---|---|
| Immunotherapy | Up-regulated Normal | Positive | Positive |
| All types of radiotherapy-immunotherapy combinations | Down-regulated | Positive | Positive or negative |
| Standard RT/ Single dose RT + immunotherapy | Up-regulated | Negative | Negative |
| Standard RT/ Single dose RT + immunotherapy | Up-regulated | Negative | Positive |
| Hypofractionated RT + immunotherapy | Down-regulated Normal | Negative | Positive |
| Hypofractionated RT + immunotherapy | Up-regulated Normal | Negative | Negative |
| Single dose RT + immunotherapy | Down-regulated | Negative | Positive |
| Single dose RT + immunotherapy | Down-regulated | Negative | Negative |

Thus in one embodiment, the method may comprise indicating immunotherapy alone as the treatment to be selected for the subject if the following are determined in the cancerous lesion compared to a control value: an increased or normal expression level of TREX1; an increased level of infiltration of immune cells; and an increased value of expression of PD-L1 on tumor cells.

In another embodiment, the method may comprise indicating radiotherapy and immunotherapy combination treatment as the treatment to be selected for the subject if the following are determined in the cancerous lesion compared to a control value: a decreased expression level of TREX1; an increased level of infiltration of immune cells; and an increased or decreased value of expression of PD-L1 on tumor cells.

In another embodiment, the method may comprise indicating standard or single dose radiotherapy and immunotherapy combination treatment as the treatment to be selected for the subject if the following are determined in the cancerous lesion compared to a control value: an increased expression level of TREX1; a decreased level of infiltration of immune cells; and an increased or decreased value of expression of PD-L1 on tumor cells.

In another embodiment, the method may comprise indicating hypofractionated radiotherapy and immunotherapy combination treatment as the treatment to be selected for the subject if the following are determined in the cancerous lesion compared to a control value: a decreased or normal expression level of TREX1; a decreased level of infiltration of immune cells; and an increased value of expression of PD-L1 on tumor cells.

In another embodiment, the method may comprise indicating hypofractionated radiotherapy and immunotherapy combination treatment as the treatment to be selected for the subject if the following are determined in the cancerous lesion compared to a control value: an increased or normal expression level of TREX1; a decreased level of infiltration of immune cells; and a decreased value of expression of PD-L1 on tumor cells.

In another embodiment, the method may comprise indicating single dose radiotherapy and immunotherapy combination treatment as the treatment to be selected for the subject if the following are determined in the cancerous lesion compared to a control value: a decreased expression level of TREX1; a decreased level of infiltration of immune cells; and an increased value of expression of PD-L1 on tumor cells.

In another embodiment, the method may comprise indicating single dose radiotherapy and immunotherapy combination treatment as the treatment to be selected for the subject if the following are determined in the cancerous lesion compared to a control value: a decreased expression level of TREX1; a decreased level of infiltration of immune cells; and a decreased value of expression of PD-L1 on tumor cells.

The input may further include data on cytosolic double stranded DNA, which may be generated in response to a specific amount of radiation dose delivered to one or more tumours in combination with a time schedule (fractionation).

The basic set of rules can be used as part of a decision support system described herein that addresses both patients with a single tumour or multiple (metastatic) setting as shown in Table 2:

**Table 2**

| | | Patient A | Patient B | | Patient C | | Patient D |
|---|---|---|---|---|---|---|---|
| | | Lesion 1 | Lesion 1 | Lesion 2 | Lesion 1 | Lesion 2 | Lesion 1 |
| Biomarker | TREX1 | Up-regulation | Normal | Up-regulation | Normal | Down-regulation/ impaired | Normal |
| | TIL | Present | Present | Negative | Negative | Present | Negative |
| | PD-L1 | Positive | Positive | Positive | Positive | Positive | Negative |
| Combinations | Hypofractionated RT + immunotherapy | + | +++ | + | +++ | +++ | +++ |
| | Single dose RT + immunotherapy | + | + | + | + | +++ | ++ |
| | Standard RT + immunotherapy | - | ++ | + | ++ | + | + |
| | Immunotherapy (mono therapy) | +++ | ++ | ++ | + | +++ | - |

### Patient A

Patient A has one lesion, with up-regulation of TREX1. As a result, the score is low in all radiotherapy modalities. Immunotherapy-only is therefore the most recommended option.

### Patient B

Patient B has two lesions. Lesion 1 has a functional TREX1 and is therefore expected to generate significant CD8+ response with hypo-fractionation. Single dose RT will cross the TREX1 threshold and therefore the correspondent score is low. In this case, irradiation of Lesion 1 with hypo-fractionation combined with a checkpoint immunotherapy agent to avoid PD-L1 up-regulation is favoured. Lesion 2 has an up-regulation of TREX1, and therefore immunotherapy-only (without radiotherapy) is the most recommended option.

### Patient C

Patient C also has two lesions, with lesion 1 having normal TREX1 function and lesion 2 having impaired TREX1 function. This would mean that lesion 1 should respond favourably to hypo-fractionation. Standard radiotherapy due to the lower dsDNA produced, is not expected to enable significant TIL recruitment in Lesion 1. Furthermore, Lesion 1 has low TILs which indicates that the TME environment may not be suitable for follow-up infiltration.

Lesion 2 has TILs present which indicates that if a sufficient CD8+ response is generated in another lesion following RT this can infiltrate the lesion. Therefore, in this case Lesion 1 and Lesion 2 have a high score for hypofractionation RT plus immunotherapy.

### Patient D

Patient D has only one lesion 1 is characterized by a low PD-L1 expression as well as low level of TILs. TREX1 function is normal. In this case, mono immunotherapy would not be recommended. Standard radiotherapy plus immunotherapy due to the immune-desert option be able to overcome the immune suppressive environment. In addition, high dose radiotherapy would be less ideal since TREX1 would cut the potential treatment immunogenicity. In this case, hypofractionation RT plus immunotherapy would be the recommended option.

The present application finds particular application in clinical decision support systems, particularly for the treatment of cancers with a combination of radiotherapy and immunotherapy. However, it will be appreciated that the described technique may also find application in other diagnostic systems, other medical scenarios, or other clinical techniques.

In one embodiment, a recommendation of what action is to be taken, e.g., by a clinician, in view of the predicted responsiveness scores, may be performed by a computer. For instance, in certain embodiments, the computer may report (i.e., generates an electronic report of) a proposed action to be taken in terms of the selection of a preferred treatment modality (e.g. radiotherapy dose and/or fractionation for use in combination with immunotherapy). The preferred radiotherapy dose and/or fractionation may in some cases be zero, e.g. the recommended treatment may comprise immunotherapy alone. In other cases the recommended action may be selection of a specific metastatic lesion in the subject for combined radio- and immunotherapy.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

Other variations of the disclosed embodiments can be understood and effected by those skilled in the art in practicing the claimed invention from a study of the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems.

## Claims

1. A computer-implemented method (200) for identifying responsiveness to therapy for a subject suffering from cancer, the method comprising:
receiving data (202) associated with a cancerous lesion in the subject;
processing the data (204) to determine a value for one or more biomarkers in the cancerous lesion; wherein the biomarkers comprise:
(i) an expression level of three prime repair exonuclease 1 (TREX1) in the cancerous lesion;
(ii) an expression level of programmed death-ligand 1 (PD-L1) on tumor cells in the cancerous lesion; and/or
(iii) a level of infiltration of immune cells in the cancerous lesion;
processing the values (206) of the biomarkers to obtain a score indicative of responsiveness of the cancerous lesion to one or more treatment modalities; wherein the treatment modalities comprise radiotherapy and immunotherapy combination treatment.

2. A method according to claim 1, wherein the method comprises receiving data associated with a plurality of different cancerous lesions in the subject; processing the data to determine values for the biomarkers in each cancerous lesion; and processing the values of the biomarkers to obtain a score indicative of responsiveness of each cancerous lesion in the subject to the treatment modalities.

3. A method according to claim 2, further comprising ranking each lesion based on the obtained scores, thereby indicating a lesion in the subject to be selected for radiotherapy and immunotherapy combination treatment.

4. A method according to any preceding claim, wherein the values of the biomarkers are processed to obtain a score indicative of responsiveness of the cancerous lesion to each of a plurality of treatment modalities; each treatment modality comprising a defined radiation dose and/or fractionation in combination with immunotherapy.

5. A method according to claim 4, further comprising ranking (208) each treatment modality based on the obtained scores, thereby indicating a radiation dose and fractionation to be selected for treatment of the subject in combination with immunotherapy.

6. A method according to any preceding claim, wherein an increased value of the expression level of TREX1 in the cancerous lesion compared to a control value is processed to obtain a score indicative of reduced responsiveness of the lesion to radiotherapy and immunotherapy combination treatment, compared to immunotherapy alone.

7. A method according to any preceding claim, wherein a reduced or unchanged value of the expression level of TREX1 in the cancerous lesion compared to the control value is processed to obtain a score indicative of increased responsiveness of the lesion to hypofractionated radiotherapy and immunotherapy combination treatment, compared to standard or single dose radiotherapy and immunotherapy combination treatment.

8. A method according to any preceding claim, wherein an increased value of the expression level of PD-L1 on tumor cells and/or a reduced level of infiltration of immune cells in the cancerous lesion compared to a control value is processed to obtain a score indicative of reduced responsiveness of the lesion to radiotherapy and immunotherapy combination treatment, compared to a lesion showing a lower value of the expression level of PD-L1 on tumor cells and/or an increased level of infiltration of immune cells.

9. A method according to any preceding claim, further comprising calculating an expected volume change of the cancerous lesion in response to the one or more treatment modalities.

10. A method according to any preceding claim, wherein the received data is derived from RNA sequencing, polymerase chain reaction (PCR), microarray analysis or immunohistochemistry of biopsy sample obtained from the subject.

11. A method according to any preceding claim, wherein the immune cells comprise tumour infiltrating lymphocytes.

12. A method according to claim 11, wherein the tumour infiltrating lymphocytes are CD8+, CD4+, FOXP3+ and/or CD3+ T cells.

13. A method according to any preceding claim, wherein the immunotherapy comprises treatment with one or more immune checkpoint inhibitors.

14. A method according to any preceding claim, the received data is derived from positron emission tomography (PET) imaging of one or more of the biomarkers in the subject.

15. A method according to any preceding claim, wherein the treatment modalities are stored in a treatment plan database; one or more treatment modalities are extracted from the database for analysis; each extracted treatment modality is analysed to determine a score indicative of responsiveness of the cancerous lesion to the treatment modality; and a treatment modality having a score indicative highest responsiveness is indicated to be selected for treatment of the subject.

16. A system (100) for identifying responsiveness to therapy for a subject suffering from cancer, the system comprising:
an interface (110) for receiving data associated with a cancerous lesion in the subject;
a memory (106); and
a processor (104) configured to execute instructions stored on the memory to:
(a) process the data to determine a value for one or more biomarkers in the sample; wherein the biomarkers comprise:
i) an expression level of three prime repair exonuclease 1 (TREX1) in the cancerous lesion;
(ii) an expression level of programmed death-ligand 1 (PD-L1) on tumor cells in the cancerous lesion; and/or
(iii) a level of infiltration of immune cells in the cancerous lesion; and
(b) process the values of the biomarkers to obtain a score indicative of responsiveness of the cancerous lesion to one or more treatment modalities; wherein the treatment modalities comprise radiotherapy and immunotherapy combination treatment.

17. A computer program product comprising a non-transitory computer readable medium, the computer readable medium having computer readable code embodied therein, the computer readable code being configured such that, on execution by a suitable computer or processor, the computer or processor is caused to perform the method of any of claims 1 to 15.
